(19)

Europäisches
Patentamt
European
Patent Office
Office européen
des brevets

(11) **EP 4 488 230 A1**

(12) **EUROPEAN PATENT APPLICATION**

(43) Date of publication:
**08.01.2025   Bulletin 2025/02**

(21) Application number: **23182975.5**

(22) Date of filing: **03.07.2023**

(51) International Patent Classification (IPC):
**C01C 1/04** *(2006.01)*        **C07C 29/151** *(2006.01)*
**C01B 3/36** *(2006.01)*        **C01B 3/38** *(2006.01)*
**C01B 3/50** *(2006.01)*        **C01B 3/48** *(2006.01)*
**C01B 3/02** *(2006.01)*

(52) Cooperative Patent Classification (CPC):
(C-Sets available)
**C01B 3/025; C01B 3/36; C01B 3/382; C01B 3/384;**
**C01B 3/48; C01C 1/0488; C07C 29/1518;**
C01B 3/50; C01B 2203/0233; C01B 2203/0244;
C01B 2203/0255; C01B 2203/0288;
C01B 2203/0405; C01B 2203/0415;
C01B 2203/043;                          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **L'AIR LIQUIDE, SOCIETE ANONYME
POUR L'ETUDE ET
L'EXPLOITATION DES PROCEDES GEORGES
CLAUDE
75007 Paris (FR)**

(72) Inventors:
• **Thiel, Matthias**
  **60439 Frankfurt am Main (DE)**
• **Schmid McGuinness, Teja**
  **60439 Frankfurt am Main (DE)**
• **Sethi, Riya**
  **60439 Frankfurt am Main (DE)**
• **Schmidt, Sophia**
  **60439 Frankfurt am Main (DE)**

(74) Representative: **Air Liquide
L'Air Liquide S.A.
Direction de la Propriété Intellectuelle
75, Quai d'Orsay
75321 Paris Cedex 07 (FR)**

(54) **PROCESS AND PLANT FOR PRODUCING METHANOL AND AMMONIA WITH REDUCED
EMISSION OF CARBON DIOXIDE**

(57)     The invention relates to a process and plant for combined production of methanol and ammonia by heterogeneously catalyzed reactions in methanol and ammonia synthesis reactors, while reducing carbon dioxide emissions. According to the invention, carbon dioxide is removed from a raw synthesis gas or shifted synthesis gas. From the carbon dioxide removed raw synthesis gas or shifted synthesis gas, different hydrogen containing gas streams are generated in a hydrogen recovery stage. The different hydrogen containing gas streams as well as the carbon dioxide removed raw synthesis gas or shifted synthesis gas can be used to substitute natural gas fuel in burners, fired heaters, and auxiliary boilers, thus reducing the carbon dioxide emissions from the process or plant.

Fig. 2

**EP 4 488 230 A1**

(52) Cooperative Patent Classification (CPC): (Cont.)
C01B 2203/046; C01B 2203/0475;
C01B 2203/061; C01B 2203/068;
C01B 2203/0822; C01B 2203/0827;
C01B 2203/1235; C01B 2203/1241;
C01B 2203/146; C01B 2203/148

C-Sets
**C07C 29/1518, C07C 31/04**

## Description

### Field of the invention

The invention relates to a process for parallel production of methanol and ammonia by heterogeneously catalyzed reaction of hydrogen and carbon oxides on the one hand and hydrogen and nitrogen on the other hand in corresponding synthesis reactors known per se, wherein the focus of the invention is on the production, conditioning and optimized material utilization of the synthesis gas required therefor. The invention further relates to a plant for performing such a production process.

### Prior art

Processes for industrial production of methanol and ammonia by heterogeneously catalyzed conversion of synthesis gas or the hydrogen present therein in suitable synthesis reactors have long been known in the art. Synthesis gases are gas mixtures containing hydrogen and carbon oxides which are used in various synthesis reactions.

Both substances constitute important indispensable feedstock chemicals of the chemical industry for further processing into end products. Ullmann's Encyclopedia of Industrial Chemistry, Sixth Edition, 1998 Electronic Release, chapter "Methanol", subchapter 5 "Process Technology" and chapter "Ammonia", subchapter 4 "Production" describes various basic processes for producing the recited substances.

A modern two-stage process for producing methanol is disclosed in European patent specification EP 0 790 226 B1 for example. The methanol is produced in a circular process wherein a mixture of fresh and partly reacted synthesis gas is supplied initially to a water-cooled reactor (WCR) and then to a gas-cooled reactor (GCR), in each of which the synthesis gas is converted over a copper-based fixed-bed catalyst to afford methanol. The methanol produced in the process is separated from the synthesis gas to be recycled which is then passed through the gas-cooled reactor in counter-current as coolant and preheated to a temperature of 220°C to 280°C before it is introduced into the first synthesis reactor. A portion of the synthesis gas to be recycled is removed from the process as a purge stream to prevent inert components from accumulating in the synthesis circuit.

Unconverted methane from synthesis gas production is considered an inert component in the context of methanol synthesis and also ammonia synthesis since this compound does not undergo further conversion under the conditions of methanol or ammonia synthesis. The same applies to argon which passes into synthesis gas production via feed streams.

A current process for ammonia synthesis is described for example in patent publication WO 2002/038499 A1. Compared to the synthesis gas used for methanol synthesis it is important in the case of synthesis gas for ammonia synthesis to completely eliminate the proportion of carbon oxides, so that hydrogen passes into the ammonia synthesis as the sole remaining synthesis gas constituent. This is effected initially through conversion of the carbon monoxide present in the synthesis gas (CO conversion), a subsequent carbon dioxide removal by means of a sorption process and finally by means of cryogenic gas fractionation.

There are different processes for producing synthesis gas comprising hydrogen and carbon oxides as input gas for methanol synthesis and ammonia synthesis, for example steam reforming, autothermal reforming (ATR), combinations thereof (so-called combined reforming) and noncatalytic partial oxidation (POX). Technical details of these processes are known in the art and are comprehensively described in, for example, Ullmann's Encyclopedia of Industrial Chemistry, Sixth Edition, 1998 Electronic Release, keyword "Gas Production".

A particularly often realized variant of steam reforming is the steam reforming of natural gas as input gas. Due to the high methane content of natural gas this is also referred to as steam methane reforming (SMR).

A further variant of steam reforming does not comprise heating the catalyst-filled reactor tubes, also known as cracking tubes, by thermal radiation using burner flames but rather comprises using hot flue gases or hot synthesis gas from a downstream reforming stage, for example from an ATR, to heat the reactor tubes in order to transfer thereto the energy required for the endothermic steam cracking. The heat transfer proceeds largely by convective means and the corresponding reformer type is known as a gas heated reformer (GHR).

Starting materials for the abovementioned processes for synthesis gas production include hydrocarbons such as natural gas, comprising its main component methane or naphtha. The recited processes afford different ratios of the product components carbon monoxide (CO) and hydrogen ($H_2$) as is apparent from the following reaction equations:

$$2\ CH_4 + O_2 \qquad\qquad = 2\ CO + 4\ H_2 \qquad \text{(partial oxidation)}$$
$$2\ CH_4 + \tfrac{1}{2}\ O_2 + H_2O \qquad = 2\ CO + 5\ H_2 \qquad \text{(autothermal reforming)}$$
$$2\ CH_4 + 2\ H_2O \qquad\quad = 2\ CO + 6\ H_2 \qquad \text{(pure steam reforming)}$$

Since partial oxidation or autothermal reforming is operated with an excess of hydrocarbon/deficiency of oxygen to inhibit the total oxidation of the hydrocarbons to carbon dioxide a synthesis gas is often obtained which has a hydrogen deficit having regard to its use as input gas for methanol synthesis. This necessitates according to the following reaction equation

$$2 \ H_2 + CO = CHsOH$$

an $H_2$/CO ratio of at least 2 and under practical synthesis conditions often slightly greater than 2, for example 2.1. This ratio is typically formulated as the stoichiometry number SN of the methanol synthesis and takes into account that carbon dioxide too reacts to afford methanol.

$$SN = ([H_2] - [CO_2]) \ / \ ([CO] + [CO_2]) \ \geq 2 \qquad (e.g. \ 2.1)$$

By contrast, synthesis gases obtained by partial oxidation or autothermal reforming often have a stoichiometry number of $\leq 1.9$, occasionally even $\leq 1.7$ auf. Accordingly, none of the reforming/partial oxidation processes in themselves afford a synthesis gas product having the stoichiometric $H_2$/CO ratio of 2 or only a slight hydrogen excess desired for the methanol synthesis.

It is moreover necessary, having regard to the ammonia synthesis to be performed in parallel, to separate carbon oxides in the proportion of synthesis gas assigned as the feed therefor and to maximize the proportion of hydrogen. This is typically effected by means of the CO conversion reaction, also known as the water gas shift reaction (WGS) or CO shift reaction, according to the reaction equation

$$CO + H_2O = CO_2 + H_2$$

Addition of steam causes the CO to react to afford $CO_2$ and $H_2$. Depending on the employed reaction temperature, the reaction is referred to as a high temperature shift (HTS), medium temperature shift (MTS) or low temperature shift (LTS).

The further workup of the produced raw synthesis gas usually also comprises a sorption process for separating further unwanted concomitants, for example by physical or chemical absorption or gas scrubbing. Such processes thus allow unwanted constituents, in particular carbon dioxide ($CO_2$), to be safely removed down to trace amounts from the desired main synthesis gas constituents hydrogen and carbon monoxide. A known and often employed process is the Rectisol process which comprises a scrubbing of the raw synthesis gas with cryogenic methanol as the absorbent and is likewise described in principle in the abovementioned document.

Cryogenic gas fractionation (so-called coldbox) may also be used to remove traces of higher hydrocarbons or of carbon monoxide. This employs mainly liquid methane or liquid nitrogen to absorb higher boiling gases such as carbon monoxide. Workup of the hydrogen required for the ammonia synthesis is typically effected by performing a liquid nitrogen scrubbing which advantageously affords a hydrogen/nitrogen mixture having the ratio ideal for ammonia synthesis of 3 mol/mol. The thus obtained offgas stream may be used as fuel gas or alternatively separated into a methane-rich gas stream and into a further carbon monoxide- and hydrogen-comprising gas stream by means of further cryogenic gas fractionation if desired or required.

A process for combined synthesis of ammonia and methanol is described for example in patent publication WO 2005/095313 A1. A disadvantage here is that the hydrogencontaining gas stream recycled to the methanol synthesis as stream 6 is withdrawn from the pure hydrogen product of the purification unit D and is therefore no longer available for the ammonia synthesis. It would also be desirable to subject further waste streams generated in this process not only to thermal utilization as fuel but rather to material utilization.

In a conventional methanol and ammonia synthesis setup, the hydrocarbon feed is first desulfurized, if required, then passed through a synthesis gas production stage which may comprise a steam reforming stage, an autothermal reforming stage (ATR), a non-catalytic partial oxidation stage (POX), or combinations of these stages. The raw synthesis gas produced therein is cooled before sending a first portion of it to a methanol synthesis loop, the methanol synthesis loop formed by one or more methanol synthesis reactors, a cooling and phase separation stage in which methanol formed is separated from unconverted synthesis gas, and recycle lines and one or more recycle compressors to recycle the unconverted synthesis gas to the one or more methanol synthesis reactors. Normally, a purge stream is branched off from the synthesis gas recycle stream to prevent accumulation of inert compounds in the synthesis gas.

From the methanol synthesis loop, the crude methanol produced is then purified in a distillation section. Depending on the stoichiometry of the reformed gas, the methanol synthesis loop might be equipped with a Hydrogen Recovery Unit (HRU) which, for example, can be a Pressure Swing Adsorption (PSA) unit or a membrane unit to adjust the desired stoichiometric number for the methanol synthesis process.

The second portion of the cooled raw synthesis gas is sent to an ammonia synthesis stage, details of which are known per se from the prior art.

Often, in the complex flowsheets used in the context of combined methanol and ammonia synthesis and work-up, additional auxiliary boilers or fired heaters are used for heating certain material streams, or for preparing steam. The boilers or heaters are operated using natural gas as fuel. The resulting flue gases from the fired heaters and auxiliary boilers are sources of direct carbon dioxide emissions.

**Description of the invention**

It is accordingly the object of the present invention to specify a process and a plant which does not exhibit the described disadvantages of the prior art and which especially makes it possible in a process for parallel production of methanol and ammonia to achieve preferably material utilization of ideally all material streams generated. The invention shall moreover make it possible to achieve an optimal adjustment of the stoichiometry number for the methanol synthesis without import of hydrogen not produced in the process.

This object is achieved in a first aspect of the invention by a process having the features of claim 1 and by a plant having the features of claim 9. Further embodiments according to further aspects of the invention are apparent from the subsidiary claims of the respective category.

Synthesis gas production conditions, methanol synthesis conditions, CO shift conditions, and ammonia synthesis conditions are to be understood as meaning the process conditions known per se to a person skilled in the art, in particular of temperature, pressure and residence time, as mentioned for example hereinabove and discussed in detail in the relevant literature and under which at least partial conversion, but preferably industrially relevant conversions of the reactants into the products of the respective process, takes place. The same applies to the choice of a suitable catalysts and suitable operating conditions thereof since in the context of the present invention all recited processes are operated under heterogeneous catalysis with the exception of partial oxidation (POX). Corresponding synthesis gas production reactors, methanol synthesis reactors, CO shift plants and ammonia synthesis reactors are known per se to those skilled in the art and described for example in the literature described at the outset.

In the context of the present disclosure, carbon dioxide removal conditions refer to the specific conditions of a selected physical or chemical carbon dioxide separation process which are known to a skilled person. A physical or chemical carbon dioxide removal process is understood to be a process that enables a fluid mixture, for example a gas mixture, to be separated into its components or undesirable components, especially carbon dioxide, to be separated from this mixture by applying suitable physicochemical conditions, for example by phase transition such as condensation or by using a suitable sorbent. When a sorption process is used, it may be based on adsorption, i.e., binding of the substance or substances to be separated to a surface or interface of the solid adsorbent, or on absorption, i.e., uptake of the substance or substances to be separated into the volume of the liquid or solid adsorbent. The substance or substances separated and bound by means of sorption are referred to as adsorbate or absorbate. The binding forces acting in this process can be of a physical or chemical nature. Accordingly, weaker, non-specific binding forces, e.g., van der Waals forces, usually act in physical sorption, whereas stronger, more specific binding forces act in chemical sorption and the adsorbate and/or absorbent is chemically changed.

As synonyms for the term absorbent, the terms solvent or washing agent in the case of liquid absorbents are used in the context of the present disclosure.

A specific, physical absorption process is the gas scrubbing with cryogenic methanol, which uses methanol as absorbent or scrubbing agent, the temperature of which has been cooled by means of cold-generating processes below ambient temperature, preferably below 0 °C, most preferably below - 30 °C. This process is known to the skilled person as the Rectisol process.

In contrast, the amine washes, which are known per se and frequently used for the absorption of carbon dioxide, are based on chemical absorption (chemisorption) and achieve high purities even at relatively low pressures in the absorption column. The selectivity is also usually higher than with physical absorption processes.

In amine washing, slightly alkaline aqueous solutions of amines, often ethanolamine derivatives, are used in an absorption unit (absorption section) usually designed as a washing column. Absorption takes place at low temperature, e.g., 40 °C, and slightly elevated pressure, e.g., 8 bara. Fresh or regenerated absorbent is fed at the top of the column and the gas stream to be separated is introduced in the lower section of the scrubbing column. In this process, carbon dioxide is reversibly chemically absorbed. The gas, which is depleted in carbon dioxide, leaves the column at the top and the loaded scrubbing agent is discharged at the bottom of the column and fed into a desorption section, which is also frequently designed as a separation column. In the desorption column (regeneration section), the reaction reverses the chemical equilibrium at a higher temperature and lower pressure, thus releasing the absorbed carbon dioxide as a gas. It can then be discharged at the head of the desorption column for further use or disposal. The absorbent regenerated in this way is returned to the absorption section.

An absorbent commonly used in amine scrubbing is methyldiethanolamine (MDEA), which is mostly used in aqueous solutions. In addition, activators, for example piperazine, are often added to accelerate carbon dioxide absorption, as described, for example, in the article "The Activator Mechanism of Piperazine in Aqueous Methyldiethanolamine Solutions," J. Ying et al, Energy Procedia 114 (2017), pp. 2078 - 2087. These mixtures are then referred to as activated MDEA solutions (aMDEA).

Cryogenic gas fractionation (so-called coldbox) may also be used to remove traces of higher hydrocarbons or of carbon monoxide. This typically employs mainly liquid methane or liquid nitrogen to absorb higher boiling gases such as carbon monoxide.

Specifically, cryogenic gas fractionation may be designed as a carbon dioxide removal method which is known per se from the prior art, for example as CryoCap process. In this context and depending on the composition of the feed gas, the cryogenic carbon dioxide removal may be used in conjunction with other separation methods like membrane separation, or pressure swing adsorption (PSA). Details of the application of the CryoCap process to carbon dioxide removal from flue gases can be found in the article "Adsorption assisted cryogenic carbon capture: an alternate path to steam driven technologies to decrease cost and carbon footprint", G. Rodrigues, M. Raventos, R. Dubet-tier, S. Ruban, 15th International Conference on Greenhouse Gas Control Technologies, GHGT-15, 15. - 18.03.2021, Abu Dhabi, UAE, pp. 1-15.

To produce pure hydrogen this is typically followed by a final step of treatment of the crude hydrogen stream in a plant for pressure swing adsorption (PSA), whose fundamental properties are set out in the book "Gasification", C. Higman and M. van der Burgt, Chapter 8.2.3 "Adsorption systems", Gulf Professional Publishing (2003). Its optimal operating pressure is between 15 and 30 bar, this then allowing hydrogen yields between 80% and 92% to be achieved. At higher operating pressures the hydrogen yield recedes. The temperature of the PSA feed gas is typically below 40 °C and any condensate formed is separated beforehand.

The pressure swing adsorption uses molecular sieves as adsorbents in a series of containers operated in a staggered cyclic mode which switches between an adsorption phase and different phases of regeneration. Regeneration of the laden adsorbent is carried out by stepwise depressurization and through the use of the gas from this operation to purge other adsorbers in the regeneration cycle at a different pressure level. Depending on the number of absorbers in a line, the hydrogen recovery may be up to 90% and up to 10%. It is possible to achieve a very high purity with about 50 ppm of argon and less than 10 ppm of other impurities.

In connection with the present invention, dividing a material stream is to be understood as meaning splitting of the stream into at least two substreams whose composition of matter and phase state correspond to that of the starting stream. By contrast, separating a material stream is to be understood as meaning splitting of the stream into at least two substreams with the aid of a phase equilibrium, wherein the compositions of the obtained material streams differ from one another and from that of the starting stream.

Liquid nitrogen scrubbing stages are known per se and described for example in Häring, H. W., Industrial Gases Processing, WILEY-VCH Verlag, Weinheim (2008), p. 156. Liquid nitrogen scrubbing stages in the context of the invention are in particular apparatuses in which by means of further cryogenic gas fractionation the obtained offgas stream is separated into a methane-rich gas stream and into a further carbon monoxide- and hydrogen-comprising gas stream which is optionally also employed in international patent application WO 2002/038499 A1.

A means is to be understood as meaning something which makes it possible to achieve, or is helpful in achieving, an objective. In particular, means for carrying out a particular process step are all physical objects which a person skilled in the art would take into consideration in order to be able to carry out this process step. For example, a person skilled in the art will consider means of introducing or discharging a material stream to include all transporting and conveying apparatuses, i.e. for example pipelines, pumps, compressors, valves and the corresponding openings in container walls which seem necessary or sensible to said skilled person for performance of this process step on the basis of his knowledge of the art.

Fluid connection between two regions or plant components is to be understood here as meaning any kind of connection that enables flow of a fluid, for example a reaction product or a hydrocarbon fraction, from one to the other of the two regions, irrespective of any interposed regions, components or required conveying means.

All approximate pressures are reported in bar as absolute pressure units, bara for short, or in gauge pressure units, barg for short, unless otherwise stated in the particular individual context.

The invention is based on the following findings:

In order to reduce the carbon dioxide emissions without compromising the energy efficiency of the overall methanol and ammonia synthesis process set-up, carbon dioxide is captured from the sources of these emissions, for example from purge gas from the methanol synthesis loop and/or ammonia synthesis loop, tail gas from a hydrogen recovery unit, distillation off gas and expansion gas obtained after flashing the raw methanol to remove solved gases before reusing these gases, e. g. by routing them to a fuel distribution manifold, so that the pressure and/or the concentration of carbon dioxide are more suited for carbon capture (CC), i. e. removal of carbon dioxide by one or more of the methods as discussed above, and a hydrogen fuel gas is generated from the raw synthesis gas which can be used to replace natural gas which is traditionally used as trim fuel, or reduce it to a minimum possible or permissible value. The hydrogen recovery unit (HRU) can be designed as a PSA or a membrane unit, or a combination of both. The use of hydrogen as fuel is carried out in auxiliary boilers as well as the in the burners of the synthesis gas production stage, or in any other burners or fired heaters.

The carbon dioxide removal technology can be an amine wash unit, a cryogenic separation unit, a physical wash stage, for example a physical wash stage using refrigerated methanol as washing agent or absorbent, or any commercially available physical or chemical carbon dioxide removal technology, the selection of which will depend technical and economic considerations like utility costs , capacity of the carbon dioxide removal unit, etc.

In detail, in order to reduce the carbon dioxide emissions, a part of the natural gas used as additional fuel or trim fuel for fired heaters or for burners in the synthesis gas production stage, and natural gas fuel for auxiliary boilers is replaced with hydrogen rich gas streams as fuel.

In order to generate this hydrogen fuel and capture carbon dioxide from the methanol synthesis loop purge gas, a part of the raw synthesis gas downstream of the synthesis gas production stage is mixed with preheated methanol synthesis loop purge gas before sending it to the water-gas shift reactor. The shift reactor can be designed as a high-temperature (HT) shift reactor, medium-temperature (MT) shift reactor, a combination of HT and low-temperature (LT) shift reactors, or an isothermal or cooled shift reactor. In the water-gas shift reactor, carbon monoxide which is present in the feed gas and steam is converted to hydrogen and carbon dioxide. In order to meet product quality requirements, a methanol wash unit, to remove methanol from the purge gas by e. g. washing with water, or from any other gas exiting the methanol synthesis loop, can be employed before sending the respective gas to the carbon dioxide removal unit.

A methanol wash unit is not necessarily required upstream of a carbon dioxide removal unit designed as a physical wash using refrigerated methanol, but in this case the use of a drying unit may be advantageous in order to minimize water ingress into the washing agent solution circulation.

Alternatively to employing a physical wash, the carbon dioxide removal unit can be designed as a cryogenic separation wherein an hydrogen rich stream is obtained for use as a fuel, and for recycling to the methanol synthesis loop in order to adjust the stoichiometric number. Optionally, also off gases obtained from the cryogenic separation, as well as a liquid or gaseous, high purity carbon dioxide product obtained therefrom, can be sent to the methanol synthesis loop as feedstock and/or sent to the synthesis gas production stage and/or partially sent to a fuel distribution system.

Optionally or alternatively, the carbon dioxide removal unit may be designed as an amine wash unit, typically operated with an activated MDEA solvent. The gas product stream obtained from the carbon dioxide removal unit may be used as hydrogen fuel for firing. A part of this carbon dioxide depleted gas may pass through the hydrogen recovery unit (HRU) in order to recycle hydrogen to the methanol synthesis loop for adjusting the stoichiometric number. Hydrogen fuel for firing can be utilized either downstream of the carbon dioxide removal unit, preferably directly after the carbon dioxide removal unit, and/or after the HRU.

The carbon dioxide removal unit, for example the amine wash unit, produces a gaseous, water saturated carbon dioxide product at low pressure. The off gas stream from the amine wash unit may be sent to auxiliary boilers, the burners of the synthesis gas production stage, or any other burners, for firing.

Similar to a chemical wash unit like an amine wash unit, a physical wash unit, for example a physical wash unit using refrigerated methanol as washing agent, may be used as carbon dioxide removal technology. The product gas from the carbon dioxide removal unit is depleted in carbon dioxide and can be used as a hydrogen fuel for firing. Again, at least a part of the carbon dioxide depleted gas may be sent through a hydrogen recovery unit to recycle hydrogen and separate inert compounds present in the methanol synthesis loop. For example, the hydrogen used for firing can be either taken directly after the carbon dioxide removal unit or from the product stream of the HRU. The carbon dioxide product stream will be at low pressure and dry when a drying unit is installed after the methanol wash unit to remove methanol from carbon dioxide. Off gases from the washing process may be sent to auxiliary boilers, the burners of the synthesis gas production stage, or any other burners, for firing.

The carbon capture technology can be a CryoCap technology in combination with a HRU (PSA or membrane or combination of both) wherein two hydrogen rich product streams with different compositions and hydrogen purities may be obtained. The purest hydrogen product stream is used for further processing, for example in a liquid nitrogen wash unit PSA as a HRU unit, in order to obtain ammonia synthesis gas as it requires a very low carbon dioxide concentration, whereas the other hydrogen product stream is used for hydrogen H2 firing in burners, fired heaters, and/or auxiliary boilers, as well as for recycling hydrogen to the methanol synthesis for adjusting the stoichiometric number. For the latter purpose, very low carbon dioxide concentrations are not required. Producing two different hydrogen rich product streams has the advantage of potentially reducing the sizes of the HRU, e. g. PSA unit, and/or the size of the carbon dioxide removal stage, e. g. the CryoCap unit. Offgases from the HRU unit and/or from the carbon dioxide removal stage can at least partially be recycled to the synthesis gas production stage, e. g. a reforming section. Additionally or alternatively, offgases from the HRU unit and/or from the carbon dioxide removal stage can at least partially be used as fuel in in any auxiliary boilers as well as any fired heaters or burners, in order to at least partially replace natural gas as fuel.

Another option is to use a amine wash unit or a physical wash, e. g. by using cold methanol, as carbon dioxide removal stage. Again, two different hydrogen rich product streams with different hydrogen purities and compositions are obtained, e. g. from different stages of a carbon dioxide removal column of the amine wash unit. In this way, the solvent recycle inside the amine wash stage is reduced. Again, the purest hydrogen product stream is used for further processing, for example in a liquid nitrogen wash unit or PSA as a HRU unit, in order to obtain ammonia synthesis gas. The second hydrogen rich stream may be withdrawn at a lower stage of the carbon dioxide removal column and has a lower hydrogen purity and a higher carbon dioxide concentration. This stream can be split into two fraction, with one fraction being routed to the HRU in order to recycle hydrogen to the methanol synthesis loop for adjusting the stoichiometric number, while the other fraction can be used to at least partially replace natural gas as fuel in any burners, fired heaters, or auxiliary boilers, or a combination of these devices.

Another source of carbon dioxide emissions may be the HRU tail gas. For an embodiment comprising a HRU and a cryogenic carbon dioxide removal unit, the HRU tail gas and/or the off gas from the cryogenic separation may be rich in unconverted methane and may be recycled to the synthesis gas production stage. For example, the methane rich gases may be recycled upstream or downstream of a prereformer. A part of the HRU tail gas or cryogenic carbon dioxide removal unit offgas may be sent to burners to purge out some inert compounds, for example methane, and thus avoid inert buildup in the methanol synthesis process. Typically, the cryogenic carbon dioxide removal unit offgas remains at high pressure during this processing, so no additional compressors are required in order to recycle this gas to the synthesis gas production stage, whereas for a carbon dioxide removal unit using an amine wash setup, some form of compression would be required.

In order to address an additional source of carbon dioxide emissions, carbon dioxide may be captured from the expansion and distillation offgas by compressing and recycling these gases to the synthesis gas production stage. When a cryogenic separation technology is employed, these gases can also be sent directly to the cryogenic separation unit.

In summary, with the invention, most of the carbon dioxide from fuel gases is captured and hydrogen rich gases are used as fuel. Thus, carbon dioxide emissions in the flue gas of the overall synthesis gas production and methanol synthesis processes are significantly reduced.

Alternatively or additionally, if hydrogen is available from electrolyzers, then this hydrogen can be used for any auxiliary boilers, the burners of the synthesis gas production stage, or any other burners, for firing. Alternatively or additionally, if hydrogen is available from electrolyzers, then this hydrogen can be used for adjusting the stoichiometric number of the synthesis gas fed to the methanol synthesis loop. This will reduce or replace the NG trim fuel and will reduce the carbon dioxide emissions.

## Particular embodiments of the invention

In a second aspect of the invention, the process according to the invention is characterized in that at least a part of the hydrogen recovery tail gas stream is introduced into the synthesis gas production stage, and converted along with the input stream containing hydrocarbons under synthesis gas production conditions. In this manner, carbonaceous components in the hydrogen recovery tail gas stream may be converted at least partially to synthesis gas products and methanol, so that the carbon utilization of the process is improved.

In a third aspect of the invention, the process according to the invention is characterized in that at least a part of the distillation offgas stream is introduced into the synthesis gas production stage, and converted along with the input stream containing hydrocarbons under synthesis gas production conditions. In this manner, carbonaceous components in the distillation offgas stream may be converted at least partially to synthesis gas products and methanol, so that the carbon utilization of the process is improved.

In a fourth aspect of the invention, the process according to the invention is characterized in that in the burners of the synthesis gas production stage and/or in a separate burner or group of burners, a fuel gas is combusted which comprises at least one element selected from the following group:

Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage. In this manner, an increased flexibility of the process is obtained with regard to selecting fuels for heating purposes.

In addition, what is advantageous here is that all of the listed fuel gases except natural gas form less carbon dioxide on combustion as compared to the combustion of natural gas. Thus, an advantageous modification of the fourth aspect of the invention comprises using natural gas as fuel gas, together with , at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage. While the carbon dioxide emission is slightly increased as compared to using a completely natural gas free fuel, the flexibility of the process is increased with regard to selecting fuels for heating purposes.

In a fifth aspect of the invention the process according to the invention is characterized in that steam is generated by vaporizing water in an auxiliary boiler by combusting a fuel gas which comprises at least one element selected from the following group:

Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage. In this manner, an increased flexibility of the process is obtained with regard to selecting fuels for heating purposes.

In a sixth aspect of the invention the process according to the invention is characterized in that at least a part of the steam which is generated by vaporizing water in the auxiliary boiler is used as the reforming steam stream and/or to operate one or more steam turbines. In this way, the import of steam as an utility and or the import of electrical energy can be reduced or avoided.

In an eighth aspect of the invention the plant according to the invention is characterized in that means are comprised that allow that at least a part of the hydrogen recovery tail gas stream is introduced into the synthesis gas production stage, and converted along with the input stream containing hydrocarbons under synthesis gas production conditions. The advantages obtained with this embodiment of the plant correspond to those obtained with the corresponding embodiment of the process.

In a ninth aspect of the invention the plant according to the invention is characterized in that means are comprised that allow that at least a part of the distillation offgas stream is introduced into the synthesis gas production stage, and converted along with the input stream containing hydrocarbons under synthesis gas production conditions. The advantages obtained with this embodiment of the plant correspond to those obtained with the corresponding embodiment of the process.

In a tenth aspect of the invention the plant according to the invention is characterized in that means are comprised that allow that in the burners of the synthesis gas production stage and/or in a separate burner or group of burners, a fuel gas is combusted which comprises at least one element selected from the following group:

Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage. The advantages obtained with this embodiment of the plant correspond to those obtained with the corresponding embodiment of the process.

In an eleventh aspect of the invention the plant according to the invention is characterized in that means are comprised that allow that steam is generated by vaporizing water in an auxiliary boiler by combusting a fuel gas which comprises at least one element selected from the following group:

Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage. The advantages obtained with this embodiment of the plant correspond to those obtained with the corresponding embodiment of the process.

In a twelfth aspect of the invention the plant according to the invention is characterized in that means are comprised that allow that at least a part of the steam which is generated by vaporizing water in the auxiliary boiler is used as the reforming steam stream and/or to operate one or more steam turbines. The advantages obtained with this embodiment of the plant correspond to those obtained with the corresponding embodiment of the process.


**Working and numerical examples**

Developments, advantages and possible applications of the invention are also apparent from the following description of working and numerical examples and the drawings. All features described and/or depicted, either in themselves or in any combination, form the invention, regardless of the way they are combined in the claims or the back-references therein.

In the figures,

Fig. 1    shows a schematic representation of the process/the plant according to a comparative example,

Fig. 2    shows a schematic representation of the process/the plant according to an embodiment of the invention.

In the following, the indication "not shown" is to be understood that an item is not shown in the discussed figure in a graphical manner.

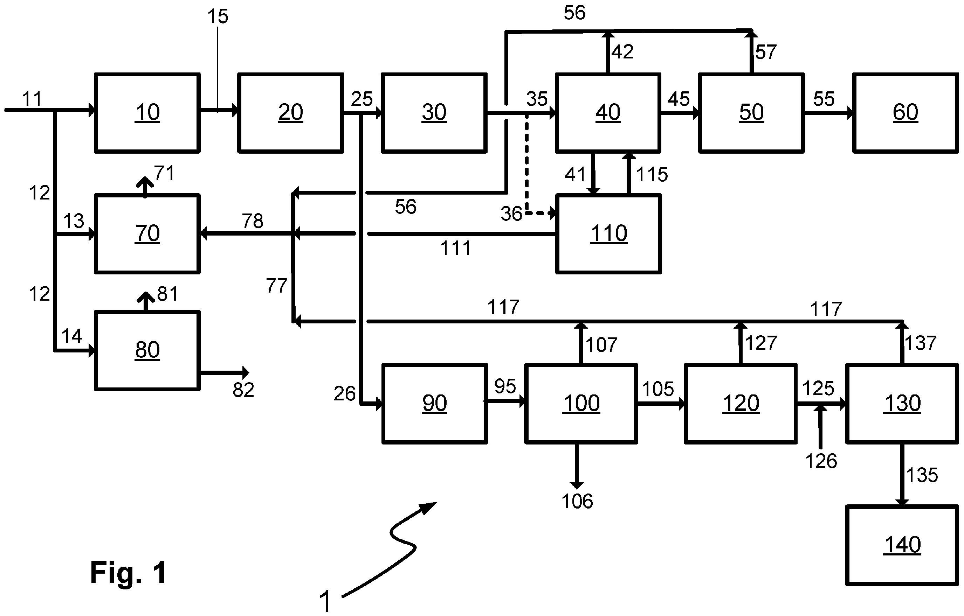

In the configuration according to the invention shown in Fig. 1, a process 1 or plant 1 for producing methanol and ammonia with reduced emission of carbon dioxide from an input stream comprising hydrocarbons is shown. In the example of Fig. 1, the input stream comprising hydrocarbons is natural gas, which is routed to a purification stage 10 via a conduit 11. Further portions of natural gas are used as fuel in one or more fired heaters or burners 70, or one or more auxiliary boilers 80. Some of the one or more fired heaters or burners 70 may be comprised in a synthesis gas production stage 20. Thus, portions of the natural gas are used as fuel are routed via conduit 11 and a conduit 13 to the one or more fired heaters or burners 70 and/or via conduit 11 and a conduit 14 to the one or more auxiliary boilers 80. Flue gases are discharged from the one or more fired heaters or burners 70 and/or from the one or more auxiliary boilers 80 via conduits 71 or 81, respectively. Further, from the one or more auxiliary boilers 80, a steam stream is discharged via a conduit 82.

In an example, the purification stage 10 comprises a desulfurization unit for removing sulfur compounds from the input stream since they may act as a catalyst poison in a downstream synthesis gas production stage.

The purified input stream is discharged from the purification stage 10 and routed to the synthesis gas production stage 20 via a conduit 15. In an example, the synthesis gas production stage is designed as a steam reforming stage, wherein the steam reforming stage comprises a plurality of reformer tubes filled with a steam reforming catalyst, arranged in a steam reformer furnace, and heated with a plurality of steam reformer burners arranged inside the steam reformer furnace, and a reforming steam stream is supplied to the steam reforming stage via a separate conduit (not shown). In an example, the synthesis gas production stage is designed as an autothermal reforming stage (ATR), wherein the autothermal reforming stage comprises an ATR reactor with an ATR burner and an ATR catalyst bed, and a reforming steam stream is supplied to the steam reforming stage via a separate conduit (not shown). In an example, the synthesis gas production stage is designed as a non-catalytic partial oxidation stage (POX), wherein the non-catalytic partial oxidation stage comprises a POX reactor with a POX burner, and an oxygen containing oxidant stream and a moderator stream comprising steam and/or carbon dioxide is supplied to the non-catalytic partial oxidation stage via separate conduits (not shown). In an example, the synthesis gas production stage comprises a combination of at least two of a steam reforming stage, an autothermal reforming stage, and a partial oxidation stage. In an example, the synthesis gas production stage comprises a prereformer (not shown) arranged upstream of any of the steam reforming stage, the autothermal reforming stage, and the partial oxidation stage, or the combination of stages.

In the synthesis gas production stage 20, the purified input stream is at least partially converted under synthesis gas production conditions to a raw synthesis gas stream containing hydrogen ($H_2$), carbon monoxide (CO) and inert components, being under methanol synthesis conditions, such as methane ($CH_4$). The raw synthesis gas stream is discharged from the synthesis gas production stage 20 via a conduit 25 and divided into a first raw synthesis gas substream and into a second raw synthesis gas substream. The first raw synthesis gas substream is introduced into a cooling stage 30. The second raw synthesis gas substream is routed to further processing steps via a conduit 26.

The first raw synthesis gas substream is cooled in the cooling stage 30 to a temperature suited for feeding the cooled first raw synthesis gas substream as a feed gas, fresh gas, or makeup gas into a methanol synthesis loop 40 (methanol synthesis stage 40) via a conduit 35. In an example, this temperature ranges between 20 and 50 °C.

In an example (not shown), the raw synthesis gas stream is divided into a first raw synthesis gas substream and into a second raw synthesis gas substream downstream of the cooling stage 30. This is advantageous if the further processing steps carried out on the second raw synthesis gas substream also require lower input temperatures.

In an example (not shown), the methanol synthesis loop 40 comprises a methanol synthesis reactor, a makeup gas compressor, a methanol synthesis feed gas feed conduit, wherein the methanol synthesis feed gas results from a combination of the makeup gas and a recycle gas, a raw methanol discharge conduit, a raw methanol cooling unit, a phase separator for separating a liquid raw methanol product stream from a recycle gas stream which contains unconverted synthesis gas constituents and inert components, notably methane, a recycle compressor for conveying the recycle gas stream back to the methanol synthesis reactor. Furthermore, as shown in Fig. 1, a purge stream conduit 41 serves for discharging a portion of the recycle gas stream from the methanol synthesis loop 40 as a purge stream, thus preventing the accumulation of inert components. These elements of the methanol synthesis loop 40 are known per se from the prior art.

The liquid raw methanol product stream is discharged from the methanol synthesis loop 40 via a conduit 45 and introduced into a methanol distillation unit 50. In the methanol distillation unit, the liquid raw methanol product stream is purified in a manner known per se from the prior art. Via a conduit 55, a purified methanol product stream is discharged from the methanol distillation unit 50 and routed to a methanol further use or storage facility 60. Moreover, from the methanol synthesis loop 40 and/or from the methanol distillation unit 50, at least one a gaseous stream is obtained and routed via a conduit 42 and/or a conduit 57, a conduit 56, a collection line 77, a conduit 78 to the one or more fired heaters or burners 70. The at least one a gaseous stream comprises a distillation offgas and an expansion gas. The distillation offgas comprises low molecular weight components retrieved from the distillation as light ends. The expansion gas comprises gases which were released from the liquid methanol phase upon pressure reduction between the methanol synthesis loop 40 and the methanol distillation unit 50, or in the methanol distillation unit 50.

Via the purge stream conduit 41, the purge gas stream from the methanol synthesis loop 40 is fed to a hydrogen recovery stage 110. In an example, the hydrogen recovery stage is designed to recover hydrogen by pressure swing adsorption. In an example, the hydrogen recovery stage is designed to recover hydrogen by membrane separation. In an example, the hydrogen recovery stage is designed to recover hydrogen by a combination of pressure swing adsorption and membrane separation.

From the hydrogen recovery stage 110, a hydrogen enriched gas stream is discharged via a conduit 115, and introduced into the methanol synthesis loop 40 in order to increase the hydrogen content of the methanol synthesis feed gas. In turn, a portion of the recycle gas stream is discharged from the methanol synthesis loop 40 via a conduit 41 as a purge stream, thus preventing the accumulation of inert components, and introducing the purge stream into the hydrogen recovery stage 110 in order to recover hydrogen from the purge stream. Optionally, a portion of the cooled first raw synthesis gas substream is branched off from conduit 35 and is introduced via a conduit 36 into the hydrogen recovery stage 110 in order to recover additional hydrogen. From the hydrogen recovery stage 110, a hydrogen recovery tail gas is discharged via a conduit 111, and routed via the collection pipe 77 and the conduit 78 to the one or more fired heaters or burners 70.

The second raw synthesis gas substream is routed in conduit 26, thus yielding a CO shift input stream. After optional cooling, the CO shift input stream is introduced into a CO shift stage 90. In an example, the CO shift stage might be designed as an integrated cooling + CO shift stage. In an example, the integrated cooling stage might be arranged upstream and/or downstream of the CO shift stage. In the CO shift stage 90, the CO shift input stream is converted under CO shift conditions into a shifted synthesis gas stream. Subsequently, the shifted synthesis gas stream is discharged from the CO shift stage, or from the integrated cooling + CO shift stage, via a conduit 95, and introduced into a carbon dioxide removal stage 100.

In the carbon dioxide removal stage 100, at least a part of the carbon dioxide is removed from the shifted synthesis gas stream by means of a physical or chemical sorption process and/or by means of a cryogenic separation process under the respective carbon dioxide removal conditions which are known per se from the prior art. In an example, the carbon dioxide removal stage 100 is designed as an amine wash stage. Via a conduit 107, a first part of the carbon dioxide depleted shifted synthesis gas stream is discharged from the carbon dioxide removal stage, and introduced into a conduit 117. Further, via a conduit 106, a carbon dioxide rich fluid stream is discharged from the carbon dioxide removal stage in gaseous or liquid form.

Via a conduit 105, a second part of the carbon dioxide depleted shifted synthesis gas stream is discharged from the carbon dioxide removal stage, and introduced into a fine purification stage 120. In an example, the fine purification stage is designed as a pressure swing adsorption stage. In an example, the fine purification stage is designed as a liquid nitrogen wash stage. In an example, the fine purification stage is designed as a combination of a pressure swing adsorption stage and a liquid nitrogen wash stage.

Via a conduit 125, a second hydrogen enriched gas stream is discharged from the fine purification stage 120 and introduced into an ammonia synthesis stage 130. Via a conduit 126, a nitrogen feed stream is introduced into the ammonia synthesis stage 130. Further, via a conduit 127, a fine purification waste gas stream is discharged from the fine purification stage 120 and introduced into a conduit 117.

In the ammonia synthesis stage 130, the second hydrogen enriched gas stream and the nitrogen feed stream are at least partially converted to an ammonia product stream under ammonia synthesis conditions. The design of the ammonia synthesis stage, and the ammonia synthesis conditions are known per se from the prior art. Via a conduit 135, the ammonia product stream is discharged from the ammonia synthesis stage 130 and introduced into an ammonia further use or storage facility 140. Further, via a conduit 137, an ammonia purge gas stream containing unconverted hydrogen and nitrogen is discharged from the ammonia synthesis stage 130 and introduced into a conduit 117.

According to the invention, the gas streams fed to conduit 117 are routed via the collection pipe 77 and the conduit 78 to the one or more fired heaters or burners 70. Alternatively (not shown), conduit 78 might be connected to exclusively or alternatively to the one or more auxiliary boilers 80 so that all or a part of the gases fed to the collection pipe 77, all containing hydrogen, are combusted in the one or more auxiliary boilers 80, thus eliminating at least a part of the natural gas fuel, and reducing the amount of carbon dioxide emitted with the flue gas in conduit 71 and/or conduit 81.

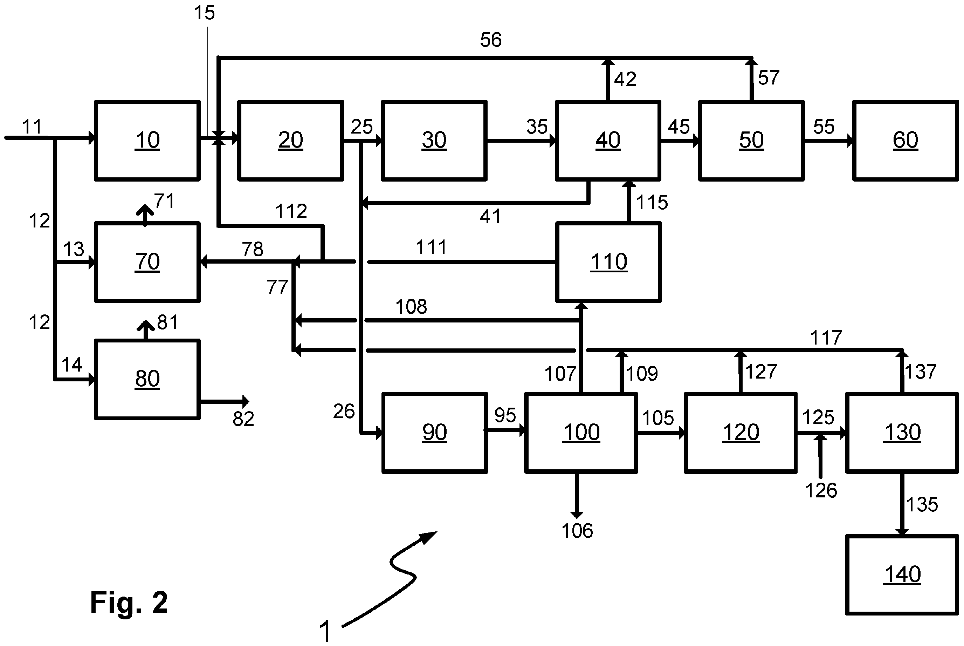

In the configuration according to the invention shown in Fig. 2, all elements as labelled with their specific reference symbols or reference numerals correspond to those elements as explained in relation to Fig. 1 with regard to their sort or kind, as well as their function and properties, unless noted otherwise in the specific context.

In contrast to Fig. 1, the gas streams in conduits 42, 57, and 56 are recycled to the synthesis gas production stage 20 via conduit 15.

Via conduit 107, a first part of the carbon dioxide depleted shifted synthesis gas stream is discharged from the carbon dioxide removal stage 100 and introduced into the hydrogen recovery stage 110 as a further feed stream. A portion of the carbon dioxide depleted shifted synthesis gas stream routed via conduit 107 is branched off and introduced into collection line 77, in order to increase the amount of fuel routed to the one or more fired heaters or burners 70 and/or the one or more auxiliary boilers 80 (conduit not shown).

Another portion of the of the carbon dioxide depleted shifted synthesis gas stream is discharged from the carbon dioxide removal stage 100 via a conduit 109 and introduced into conduit 117.

At least a part of the hydrogen recovery tail gas stream, being discharged from the hydrogen recovery unit 110 via conduit 111, is recycled to the synthesis gas production stage via a conduit 112 and conduit 15.

Via the purge stream conduit 41, the purge gas stream from the methanol synthesis loop 40 is introduced into conduit 26, thus adding up to the CO shift feed stream, rather than routing it to the hydrogen recovery stage 110 as shown in Fig. 1.

With the embodiment of the invention as shown in Fig. 2, the amount of hydrogen fed to the one or more fired heaters or burners 70 and/or the one or more auxiliary boilers 80 is increased in comparison to Fig. 1, and thus the carbon dioxide emission of the combined methanol and ammonia synthesis process is further reduced.

Numerical examples

The following example is based on a combined 5000 metric ton per day methanol plant + 3000 metric ton per day ammonia plant with a common synthesis gas production stage (ATR based reforming) from natural gas. The approach was to reduce carbon dioxide emissions to the atmosphere as much as possible. Captured carbon dioxide can be used for sequestration. The combined synthesis plant was designed to be self-sufficient (own power generation), only electrical power import for start-up was considered. In the carbon dioxide removal stage, an amine wash unit was employed.

A selection of performance parameters for different design options are summarized in the following table:

|  | Case 1 | Case 2 | Case 3 | Case 4 |
|---|---|---|---|---|
| $CO_2$ emissions from fired heater and auxiliary boiler [kg/h]* | 125180 | 107783 | 66943 | 62001 |
| $CO_2$ capture rate [%] | 56.9 | 63.0 | 78.5 | 80.4 |
| Residual carbon footprint [kg $CO_2$ emitted/(kg MeOH + kg NH3)] | 0.37 | 0.32 | 0.20 | 0.18 |
| Energy intensity [GJ/(t MeOH + t NH3)] | 31.04 | 31.06 | 32.16 | 32.45 |

*Direct emissions only Case 1: Conventional setup, no interconnection between methanol synthesis and ammonia synthesis, PSA included in methanol synthesis unit (comparative example).

Case 2: Purge gas of methanol synthesis is routed to ammonia synthesis (CO shift). Part of product outlet of $CO_2$ removal unit is routed to a PSA. PSA hydrogen rich outlet stream is routed to methanol synthesis.

Case 3: Same as Case 2, but more natural gas is processed and routed to the ammonia synthesis. Hydrogen rich gas from the outlet of $CO_2$ removal unit is used for hydrogen firing in fired heater and aux boiler.

Case 4: Same as Case 3. In addition, a portion of PSA tail gas is compressed again and routed to the synthesis gas production stage (reforming section). (Expansion gas and Distillation off-gas are not included in the recycle.)

Case 4 partially represents figure 2. In this case, conduit 56 that joins conduit 15 is not included. It joins the fuel header conduit 78.

A plant design according to Case 1 leads to the highest carbon dioxide emissions to the atmosphere and the lowest carbon dioxide capture rate. Consequently, Case 1 has the highest residual carbon footprint. Routing the purge gas of the methanol synthesis to the CO-shift units of the ammonia synthesis and providing the lack of hydrogen for right stoichiometric number in the methanol synthesis by routing the hydrogen rich stream from the outlet of the carbon dioxide removal stage via a PSA to the methanol synthesis unit reduces the total carbon dioxide emissions as more carbon dioxide is captured in the carbon dioxide removal unit. The carbon rich off gas stream from the PSA is reduced which is combusted in the fired heater, thus leading to more carbon dioxide emissions. Consequently, the residual carbon foot print is lower.

Processing more natural gas to provide a hydrogen rich gas from the outlet of the carbon dioxide removal unit as a fuel for fired heaters and/or auxiliary boilers in order to minimize natural gas firing has a major impact on carbon dioxide emissions as much more carbon dioxide is captured. The carbon atoms in the hydrocarbons of the natural gas are converted via reforming and shift reactions into hydrogen and carbon dioxide from which the latter is captured in the carbon dioxide removal unit and not emitted to the atmosphere as it would be if the hydrocarbon were combusted directly in the fired heaters and/or auxiliary boilers. The less emitted carbon dioxide leads to a significantly lower residual carbon footprint. However, the energy intensity increases as more natural gas needs to be processed to convert it to hydrogen and carbon dioxide, which requires more energy than just combusting natural gas in fired heaters and/or auxiliary boilers.

Carbon dioxide emissions, carbon capture rate and residual carbon footprint improve further if a part of the PSA tail gas is recompressed and fed to the reforming section.

The PSA tailgas still consists of a significant fraction of methane. This methane can be reformed again instead of firing it as a fuel in the fired heaters and/or auxiliary boilers. There is however a limit in the amount of PSA tail gas which can be recycled to the reforming section as a higher portion of inert components will accumulate, thus leading to a lower efficiency of the process. Compressing the low pressure PSA tailgas to the process pressure of the reforming section requires additional energy, leading to a higher energy intensity.

**List of reference symbols**

| | |
|---|---|
| [1] | Methanol and ammonia production process or plant |
| [10] | Purification stage |
| [11] | Conduit |
| [12] | Conduit |
| [13] | Conduit |
| [14] | Conduit |
| [15] | Conduit |
| [20] | Synthesis gas production stage |
| [25] | Conduit |
| [26] | Conduit |
| [30] | Cooling stage |
| [35] | Conduit |
| [36] | Conduit |
| [40] | Methanol synthesis loop (methanol synthesis stage) |
| [41] | Conduit |
| [42] | Conduit |
| [45] | Conduit |
| [50] | Methanol distillation unit |
| [55] | Conduit |
| [56] | Conduit |
| [57] | Conduit |
| [60] | Methanol further use or storage facility |
| [70] | One or more fired heaters or burners |
| [71] | Conduit |
| [77] | Conduit (collection line) |
| [78] | Conduit |
| [80] | One or more auxiliary boilers |
| [81] | Conduit |
| [82] | Conduit |
| [90] | CO shift stage |
| [95] | Conduit |
| [100] | Carbon dioxide removal stage |
| [105] | Conduit |
| [106] | Conduit |
| [107] | Conduit |
| [108] | Conduit |
| [109] | Conduit |
| [110] | Hydrogen recovery stage (HRU) |
| [111] | Conduit |
| [112] | Conduit |
| [115] | Conduit |
| [117] | Conduit |
| [120] | Fine purification stage |
| [125] | Conduit |
| [126] | Conduit |
| [127] | Conduit |
| [130] | Ammonia synthesis loop (ammonia synthesis stage) |
| [135] | Conduit |
| [137] | Conduit |
| [140] | Ammonia further use or storage facility |

**Claims**

**1.** Process for producing methanol and ammonia with reduced emission of carbon dioxide from an input stream comprising hydrocarbons, preferably methane, ethane, or naphtha, comprising the following steps of:

(a) Providing the input stream containing hydrocarbons and a reforming steam stream;
(b) supplying the input stream containing hydrocarbons to a synthesis gas production stage, the synthesis gas production stage comprising one or more elements form the following group:

(b1) a steam reforming stage, wherein the steam reforming stage comprises a plurality of reformer tubes filled with a steam reforming catalyst, arranged in a steam reformer furnace, and heated with a plurality of steam reformer burners arranged inside the steam reformer furnace,
supplying a reforming steam stream to the steam reforming stage;
(b2) an autothermal reforming stage (ATR), wherein the autothermal reforming stage comprises an ATR reactor with an ATR burner and an ATR catalyst bed,
supplying a reforming steam stream and an oxygen containing oxidant stream to the autothermal reforming stage;
(b2) a non-catalytic partial oxidation stage (POX), wherein the non-catalytic partial oxidation stage comprises a POX reactor with a POX burner,
supplying an oxygen containing oxidant stream and optionally supplying a moderator stream comprising steam and/or carbon dioxide to the non-catalytic partial oxidation stage;

(c) at least partially converting the input stream containing hydrocarbons in the synthesis gas production stage under synthesis gas production conditions to afford a raw synthesis gas stream containing hydrogen ($H_2$), carbon monoxide (CO) and inert components, being inert under methanol synthesis conditions, such as methane ($CH_4$);
(d) discharging a raw synthesis gas stream from the synthesis gas production stage and dividing the raw synthesis gas stream into a first raw synthesis gas substream and into a second raw synthesis gas substream;
(e) introducing at least a portion of the first raw synthesis gas substream into a methanol synthesis stage, at least partially converting the first raw synthesis gas substream in the methanol synthesis stage under methanol synthesis conditions;
(f) discharging a raw methanol product stream and a methanol purge gas stream containing unconverted synthesis gas constituents and inert components from the methanol synthesis stage;
(g) introducing the raw methanol product stream into a methanol distillation stage,

separating the raw methanol product stream under methanol distillation conditions into a final liquid methanol product stream, at least one liquid side product stream, and a distillation offgas stream which comprises expansion gas,
discharging the final liquid methanol product stream, the at least one liquid side product stream, and the distillation offgas stream from the methanol distillation stage;

(h) combining at least a portion of the second raw synthesis gas substream with at least a portion of the methanol purge gas stream to a CO shift input stream,

introducing the CO shift input stream into a CO shift stage,
converting the CO shift input stream in the CO shift stage under CO shift conditions into a shifted synthesis gas stream,
discharging the shifted synthesis gas stream from the CO shift stage;

(i) introducing the shifted synthesis gas stream into a carbon dioxide removal stage,

wherein the carbon dioxide removal stage is designed to remove carbon dioxide by means of a physical or chemical sorption process and/or by means of a cryogenic separation process,
removing at least a part of the carbon dioxide from the shifted synthesis gas in the carbon dioxide removal stage under carbon dioxide removal conditions,
discharging a carbon dioxide depleted shifted synthesis gas stream and a carbon dioxide rich fluid stream from the carbon dioxide removal stage;

(j) introducing a first part of the shifted synthesis gas stream and/or the carbon dioxide depleted shifted synthesis gas stream into a hydrogen recovery stage,

wherein the hydrogen recovery stage is designed to recover hydrogen by pressure swing adsorption and/or membrane separation,
discharging a first hydrogen enriched gas stream and a hydrogen recovery tail gas stream from the hydrogen recovery stage;

(k) introducing at least a part of the first hydrogen enriched gas stream into the methanol synthesis stage;
(l) introducing a second part of the shifted synthesis gas stream and/or the carbon dioxide depleted shifted synthesis gas stream into a fine purification stage,

wherein the fine purification stage is designed as a pressure swing adsorption stage and/or as a liquid nitrogen wash stage,
discharging a second hydrogen enriched gas stream and a fine purification waste gas stream from the fine purification stage;

(m) introducing at least a portion of the second hydrogen enriched gas stream and a nitrogen feed stream into an ammonia synthesis stage, at least partially converting the second hydrogen enriched gas stream and the nitrogen feed stream in the ammonia synthesis stage under ammonia synthesis conditions;
(n) discharging an ammonia product stream and ammonia purge gas stream containing unconverted hydrogen and nitrogen from the ammonia synthesis stage.

2. Process according to claim 1, **characterized in that** at least a part of the hydrogen recovery tail gas stream is introduced into the synthesis gas production stage, and converted along with the input stream containing hydrocarbons under synthesis gas production conditions.

3. Process according to claim 1 or 2, **characterized in that** at least a part of the distillation offgas stream is introduced into the synthesis gas production stage, and converted along with the input stream containing hydrocarbons under synthesis gas production conditions.

4. Process according to any of the preceding claims, **characterized in that** in the burners of the synthesis gas production stage and/or in a separate burner or group of burners, a fuel gas is combusted which comprises at least one element selected from the following group:
Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage.

5. Process according to any of the preceding claims, **characterized in that** steam is generated by vaporizing water in an auxiliary boiler by combusting a fuel gas which comprises at least one element selected from the following group:
Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage.

6. Process according to claim 5, **characterized in that** at least a part of the steam which is generated by vaporizing water in the auxiliary boiler is used as the reforming steam stream and/or to operate one or more steam turbines.

7. Plant for producing methanol and ammonia with reduced emission of carbon dioxide from an input stream comprising hydrocarbons, preferably methane, ethane, or naphtha, comprising the following constituents, being in fluid connection with each other:

(a) Means for providing the input stream containing hydrocarbons and a reforming steam stream;
(b) a synthesis gas production stage, means for supplying the input stream containing hydrocarbons to the synthesis gas production stage, the synthesis gas production stage comprising one or more elements form the following group:

(b1) a steam reforming stage, wherein the steam reforming stage comprises a plurality of reformer tubes filled with a steam reforming catalyst, arranged in a steam reformer furnace, and heated with a plurality of steam reformer burners arranged inside the steam reformer furnace,
means for supplying a reforming steam stream to the steam reforming stage;

(b2) an autothermal reforming stage (ATR), wherein the autothermal reforming stage comprises an ATR reactor with an ATR burner and an ATR catalyst bed,

means for supplying a reforming steam stream and an oxygen containing oxidant stream to the autothermal reforming stage;

(b3) a non-catalytic partial oxidation stage (POX), wherein the non-catalytic partial oxidation stage comprises a POX reactor with a POX burner,

means for supplying an oxygen containing oxidant stream and optionally supplying a moderator stream comprising steam and/or carbon dioxide to the non-catalytic partial oxidation stage;

(c) means for discharging a raw synthesis gas stream from the synthesis gas production stage and means for dividing the raw synthesis gas stream into a first raw synthesis gas substream and into a second raw synthesis gas substream;

(d) a methanol synthesis stage, means for introducing at least a portion of the first raw synthesis gas substream into the methanol synthesis stage;

(e) means for discharging a raw methanol product stream and a methanol purge gas stream containing unconverted synthesis gas constituents and inert components from the methanol synthesis stage;

(f) a methanol distillation stage, means for introducing the raw methanol product stream into the methanol distillation stage,

means for separating the raw methanol product stream under methanol distillation conditions into a final liquid methanol product stream, at least one liquid side product stream, and a distillation offgas stream which comprises expansion gas,

means for discharging the final liquid methanol product stream, the at least one liquid side product stream, and the distillation offgas stream from the methanol distillation stage;

(g) means for combining at least a portion of the second raw synthesis gas substream with at least a portion of the methanol purge gas stream to a CO shift input stream,

means for introducing the CO shift input stream into a CO shift stage,

means for discharging the shifted synthesis gas stream from the CO shift stage;

(h) a carbon dioxide removal stage, means for introducing the shifted synthesis gas stream into the carbon dioxide removal stage,

wherein the carbon dioxide removal stage is designed to remove carbon dioxide by means of a physical or chemical sorption process and/or by means of a cryogenic separation process,

means for discharging a carbon dioxide depleted shifted synthesis gas stream and a carbon dioxide rich fluid stream from the carbon dioxide removal stage;

(i) a hydrogen recovery stage, means for introducing a first part of the shifted synthesis gas stream and/or the carbon dioxide depleted shifted synthesis gas stream into the hydrogen recovery stage,

wherein the hydrogen recovery stage is designed to recover hydrogen by pressure swing adsorption and/or membrane separation,

means for discharging a hydrogen enriched gas stream and a hydrogen recovery tail gas stream from the hydrogen recovery stage;

(j) means for introducing at least a part of the hydrogen enriched gas stream into the methanol synthesis stage.

(k) a fine purification stage, means for introducing a second part of the shifted synthesis gas stream and/or the carbon dioxide depleted shifted synthesis gas stream into the fine purification stage,

wherein the fine purification stage is designed as a pressure swing adsorption stage and/or as a liquid nitrogen wash stage,

means for discharging a second hydrogen enriched gas stream and a fine purification waste gas stream from the fine purification stage;

(l) an ammonia synthesis stage, means for introducing at least a portion of the second hydrogen enriched gas stream and a nitrogen feed stream into the ammonia synthesis stage;

(m) means for discharging an ammonia product stream and ammonia purge gas stream containing unconverted hydrogen and nitrogen from the ammonia synthesis stage.

8. Plant according to claim 7, **characterized in that** means are comprised that allow that at least a part of the hydrogen recovery tail gas stream is introduced into the synthesis gas production stage, and converted along with the input stream containing hydrocarbons under synthesis gas production conditions.

9. Plant according to claim 7 or 8, **characterized in that** means are comprised that allow that at least a part of the distillation offgas stream is introduced into the synthesis gas production stage, and converted along with the input stream containing hydrocarbons under synthesis gas production conditions.

10. Plant according to any of claims 7 to 9, **characterized in that** means are comprised that allow that in the burners of the synthesis gas production stage and/or in a separate burner or group of burners, a fuel gas is combusted which comprises at least one element selected from the following group:
Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage.

11. Plant according to any of claims 7 to 10, **characterized in that** means are comprised that allow that steam is generated by vaporizing water in an auxiliary boiler by combusting a fuel gas which comprises at least one element selected from the following group:
Natural gas, at least a part of the first hydrogen enriched gas stream, at least a part of the second hydrogen enriched gas stream, at least a part of the hydrogen recovery tail gas stream, at least a part of the shifted synthesis gas stream, at least a part of the carbon dioxide depleted shifted synthesis gas stream, at least a part of the fine purification waste gas stream, at least a part of the ammonia purge gas stream, hydrogen generated in a water electrolysis stage.

12. Plant according to any of claims 7 to 11, **characterized in that** means are comprised that allow that at least a part of the steam which is generated by vaporizing water in the auxiliary boiler is used as the reforming steam stream and/or to operate one or more steam turbines.

Fig. 1

**Fig. 2**

| | Europäisches Patentamt European Patent Office Office européen des brevets | **EUROPEAN SEARCH REPORT** | Application Number EP 23 18 2975 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 7 521 483 B2 (LURGI AG [DE]) 21 April 2009 (2009-04-21) * claims 1-2 * * abstract * * column 2, line 5 – column 4, line 67 * | 1-12 | INV. C01C1/04 C07C29/151 C01B3/36 C01B3/38 C01B3/50 C01B3/48 C01B3/02 |
| Y | US 8 247 463 B2 (YOSHIDA NOBUHIRO [JP]; HIROTANI KUNIO [JP]; TOYO ENGINEERING CORP [JP]) 21 August 2012 (2012-08-21) * abstract * * figure 1 * * claims 1-4 * * column 6, line 31 – column 9, line 9 * | 1-12 | |
| Y | US 2022/315434 A1 (WALTER STEFAN [DE] ET AL) 6 October 2022 (2022-10-06) * paragraph [0042] * * paragraph [0073] – paragraph [0074] * * paragraph [0078] – paragraph [0081] * * figure 1 * * claims 1,9 * | 1-12 | |
| | | | TECHNICAL FIELDS SEARCHED (IPC) |
| Y | DK 2021 00461 A1 (HALDOR TOPSOE AS [DK]) 1 February 2022 (2022-02-01) * claim 1 * * paragraph [0120] * * paragraph [0124] * * paragraph [0142] * | 2-6,8-12 | C07C C01C C01B |

The present search report has been drawn up for all claims

| Place of search | Date of completion of the search | Examiner |
|---|---|---|
| Munich | 9 January 2024 | Alvarez Rodriguez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 18 2975**

This annex lists the patent family members relating to the patent documents cited in  the above-mentioned European search report.
The members are as contained in the European Patent Office EDP file on
The European Patent Office is in no way liable for these particulars which are merely given for the purpose of information.

**09-01-2024**

| Patent document cited in search report | | Publication date | Patent family member(s) | | Publication date |
|---|---|---|---|---|---|
| US 7521483 | B2 | 21-04-2009 | AT | E450491 T1 | 15-12-2009 |
| | | | DE | 102004014292 A1 | 20-10-2005 |
| | | | EP | 1751080 A1 | 14-02-2007 |
| | | | US | 2007299144 A1 | 27-12-2007 |
| | | | WO | 2005095313 A1 | 13-10-2005 |
| US 8247463 | B2 | 21-08-2012 | AT | E545623 T1 | 15-03-2012 |
| | | | DK | 2196448 T3 | 29-05-2012 |
| | | | EP | 2196448 A1 | 16-06-2010 |
| | | | JP | 5355062 B2 | 27-11-2013 |
| | | | JP | 2010138051 A | 24-06-2010 |
| | | | US | 2010150810 A1 | 17-06-2010 |
| US 2022315434 | A1 | 06-10-2022 | AU | 2022201856 A1 | 20-10-2022 |
| | | | CA | 3153157 A1 | 30-09-2022 |
| | | | EP | 4066921 A1 | 05-10-2022 |
| | | | US | 2022315434 A1 | 06-10-2022 |
| DK 202100461 | A1 | 01-02-2022 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0790226 B1
- WO 2002038499 A1
- WO 2005095313 A1

### Non-patent literature cited in the description

- Methanol. Ullmann's Encyclopedia of Industrial Chemistry. 1998
- Ammonia. ULLMANN'S ENCYCLOPEDIA OF INDUSTRIAL CHEMISTRY. 1998
- Gas Production. Ullmann's Encyclopedia of Industrial Chemistry. 1998
- **J. YING et al.** The Activator Mechanism of Piperazine in Aqueous Methyldiethanolamine Solutions. *Energy Procedia*, 2017, vol. 114, 2078-2087
- **G. RODRIGUES** ; **M. RAVENTOS** ; **R. DUBET-TIER** ; **S. RUBAN**. Adsorption assisted cryogenic carbon capture: an alternate path to steam driven technologies to decrease cost and carbon footprint. *15th International Conference on Greenhouse Gas Control Technologies*, 18 March 2021, 1-15
- Adsorption systems. **C. HIGMAN** ; **M. VAN DER BURGT**. Gasification. Gulf Professional Publishing, 2003
- **HÄRING, H. W.** Industrial Gases Processing. WILEY-VCH Verlag, 2008, 156